(19) Europäisches Patentamt
European Patent Office
Office européen des brevets

(11) EP 1 669 379 B1

(12) **EUROPEAN PATENT SPECIFICATION**

(45) Date of publication and mention of the grant of the patent:
**14.11.2007 Bulletin 2007/46**

(21) Application number: **03811282.7**

(22) Date of filing: **23.09.2003**

(51) Int Cl.:
***C08F 220/04*** *(2006.01)* ***C08F 120/06*** *(2006.01)*
***C08F 126/02*** *(2006.01)* ***C08F 216/06*** *(2006.01)*
***C08F 226/02*** *(2006.01)* ***C08F 226/10*** *(2006.01)*
***A61K 31/13*** *(2006.01)* ***A61K 47/32*** *(2006.01)*
***A61P 31/12*** *(2006.01)*

(86) International application number:
**PCT/RU2003/000417**

(87) International publication number:
**WO 2005/028528 (31.03.2005 Gazette 2005/13)**

(54) **3,5-DIMETHYL-1-ADAMANTYL-AMMONIUM POLYMERIC SALTS AND THE USE THEREOF IN THE FORM OF ANTIVIRAL AGENTS**

POLYMERE 3,5-DIMETHYL-1-ADAMANTYLAMMONIUMSALZE UND DEREN VERWENDUNG IN FORM VON VIRUSTATIKA

SELS POLYMERIQUES DE 3,5-DIMETHYL-1-ADAMANTYL-AMMONIUM ET LEUR UTILISATION EN TANT QU'AGENTS ANTIVIRAUX

(84) Designated Contracting States:
**AT BE BG CH CY CZ DE DK EE ES FI FR GB GR HU IE IT LI LU MC NL PT RO SE SI SK TR**

(43) Date of publication of application:
**14.06.2006 Bulletin 2006/24**

(73) Proprietor: **Akciju Sabiedriba "Olainfarm"**
**2114 Olaine (LV)**

(72) Inventors:
- **MARCHUKOV, Valery Aleksandrovich**
  **St.Petersburg, 193076 (RU)**
- **PLATONOV, Vitaly Georgievich**
  **St. Petersburg, 195269 (RU)**
- **SELKOV, Sergei Alekseevich**
  **St. Petersburg, 197198 (RU)**
- **CHERNOBROVY, Aleksandr Nikolaevich**
  **LV-2114 Olaine (LV)**

(74) Representative: **Patentanwälte Zellentin & Partner**
**Innere Wiener Strasse 8**
**81667 München (DE)**

(56) References cited:
**RU-C1- 2 071 323** **US-A- 5 726 284**
**US-A1- 2004 241 580** **US-B2- 6 517 847**

EP 1 669 379 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

### Field of the invention

This invention relates to the synthesis of new compounds of 3.5-dimethyl-1-adamantylammonium and specifically to the polymeric salts of 3.5-dimethyl-1-adamantylammonium and carboxylated (co)polymers, as well as to use of produced salts as antiviral medications.

### Background of the invention

Some compounds of amino derivatives from the adamantan row are known to possess biological activity, in particular, they are used to treat Parkinson's disease, and have antiviral effect on certain kinds of viruses.

There is a MEMANTINE (akatinol) medication which is hydrochloride 3.5-dimethyl-1-adamantylamine (HC-DMAAM) recommended for treatment of Parkinson's disease. However, its quick excretion from an organism requires its frequent intake or a dose increase which causes toxic effect on an organism (M.D. Mashkovski "Medicinal Agents", in 2 volumes, M, "New Wave". 2002, vol.1, p.143).

MIDANTAN medication which is hydrochloride 1-adamatilamine is used for treatment of Parkinsonism. Also it shows antiviral affect against the virus of the influenza of A type (ibid, vol.1, p.142). The medication as well as memantine are effective when taken frequently and constantly in therapeutic doses and do not have any prophylactic effect. A dose increase and long course of midantan intake causes unfavorable side effects.

Among the amino derivatives of adamantan, REMANTADIN, i.e. hydrochloride α-methyl-α-(1-adamantyl)methylamine, is efficient against the virus of the A type influenza. However, this antiviral agent also does not have any prophylactic effect and is effective when taken constantly and many times during treatment (ibid, vol.2, p. 325).

The above review of the biological properties of the amino derivatives of adamantan (memantine, midantan, remantadin) shows that even a slight structural change of these homotypes and isomers causes significant and complex changes of their properties which are unobvious for a specialist in advance.

It is also known that the effectiveness of therapeutic action of a physiologically active substance as a result of its adjoining to a polymer can change considerably. Production of polymer structure of a physiologically active substance may cause appearance of new properties, enhancement of the existing ones as well as loss of physiological activity.

Thus, the antibacterial activity of the product of kanamycin interaction with dialdehyde dextran where an antibiotic is adjoined by a covalent link is close to the activity of an initial kanamycin. At the same time ampicillin adjoining to dealdehyde dextran is accompanied by a considerable (10 - 15 times) decrease of antibiotic activity. A different degree of the decrease in the activity of the antibiotics kanamicine and ampicillin when they are adjoined to dialdehyde dextran depends on the influence of the structure of a polymer molecule on the hydrolytic resistance of linking, even of one type (V.A. Snezhko, L.N. Samoilova et al. - Antibiotics Journal, 1972, vol.17, №1, p.48 - 52).

In a series of cases polymer analogs can lose their biological properties characteristic for low-molecular structures. Thus, e.g. poly-2-vinylpiridine-N-oxide and some other N-oxides are well known for their antisilicosis activity; at the same time polymers containing N-oxide group screened by an alkylene radical do not feature this activity. (E.F. Razvodovski in col. Progress of Polymer Chemistry and Physics, M, Chemistry, 1973, p.305).

It was found that some polymeric compounds of amino derivatives of adamantan as compared to low-molecular analogs have longer (prolonged) physiological action and a wider range of biological activity.

The analysis of known developments, which are partially listed below, shows that search and exploration of new polymeric compounds in the row of the amino derivatives of adamantan are prospective for significant enhancement of biological properties as compared to the initial low-molecular amino compound, for extension of its efficacy time and expansion of a biological activity range. However, unfortunately, till now this search has been based not on an accurate theoretical analysis but on a researcher's intuition and numerous tests.

Rather fortunate findings were, for example, polymeric compounds of adamantylamine (USSR, author's sertificate №523618, Mcl. C08F 8/32, 1975) and polymeric salts of α-methyl-α-(1-adamantyl) methylamine (USSR, №640544, Mcl. C08F 220/04, C08F 8/32, 1978). In particular, based on a vinyl alcohol copolymer with N-vinylamidosuccinic acid and α-methyl-α-(1-adamantyl)methylamine, their polymeric salt was produced, which is a substance of the POLYREM medication. The polymeric medication POLYREM has a prolonged action against viruses of herpes of types I and 11, tick-borne encephalitis and A type influenza virus. It shows an antitoxic property, induces interferon and has an immunopotentiating effect. The medication is mainly used for prevention and treatment of the said viral infections (patent of the RF №2071323, Mcl. 6 A61 K31/785; A61K 9/06, 9/20 "Antiviral medication POLYREM" published on 10.01.1997; F.I. Ershov Antiviral Medications, M, Medicine, 1998, p. 141 -142). This medication (POLYREM) is the closest to said compounds by its chemical composition. That is why it is selected as a prototype.

## Objects of the invention

It is an object of the invention to synthesize new compounds of 3.5-dimethyl-1-adamantylammonium and specifically salts of 3.5-dimethyl-1-adamantylammonium and carboxylated (co)polymers, and to identify the compounds with antiviral activity and durable action among them.

## Task solution (Detailed description of the invention)

The determined task as regards new substances is solved through the synthesis of salts of 3.5-dimethyl-1-adamantylammonium and water-soluble oxylated copolymers of a general formula (1),

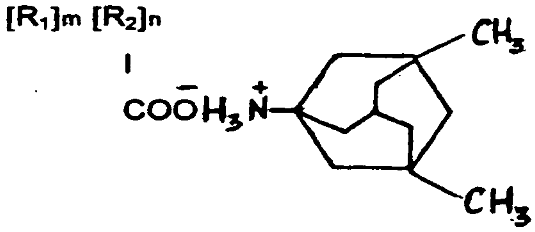

where **m** and **n -** mol fractions, **m = 100 - n**,
and carboxylated copolymers are selected from the group:

polyvinylaminosuccinic acid;

copolymer of vinyl alcohol and N-vinylaminosuccinic acid,

copolymer of N-vinylpyrrolidone and N-vinylaminosuccinic acid;

copolymer of N-vinylpyrrolidone and crotonic acid;

copolymer of N-vinylaminosuccinimide and maleic anhydride;

copolymer of N-vinylpyrrolidone and maleic anhydride;

polyacrylic acid.

1) Polymer - polyvinylaminosuccinic acid (PVASA)

$R_1 = -CH_2-CH-$ (with side chain $|$ $NH$ $|$ $CO-(CH_2)_2-COOH$) ; $R_2 = -CH_2-CH$ (with side chain $|$ $NH$ $|$ $CO-(CH_2)_2-$) ; $n = 5 - 100$

2) Copolymer of vinyl alcohol and N-vinylaminosuccinic acid (VA)m - (VASA)n

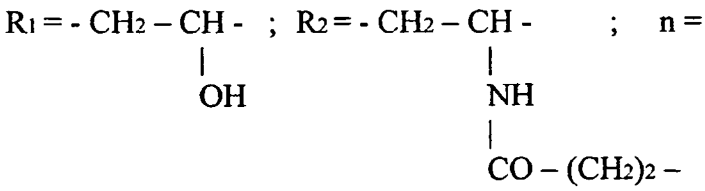

; n = 1 – 99, better 5 - 50

3) Copolymer of N-vinylpyrrolidone and N-vinylaminosuccinic acid (VP)m - (VASA)n

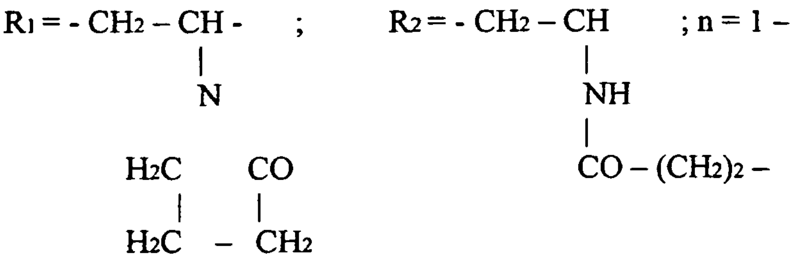

; n = 1 – 99, better 5 - 50

4) Copolymer of N-vinylpyrrolidone and crotonic acid (VP)m - (CA)n

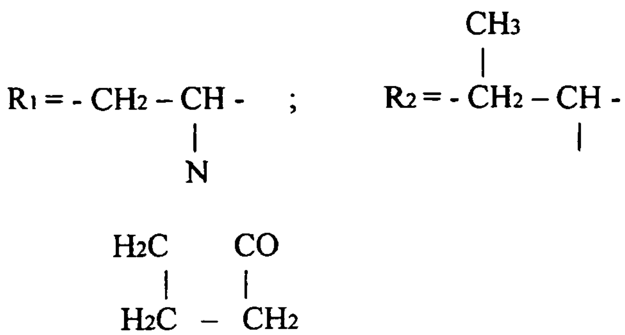

; n = 5 - 15

5) Copolymer of N-vinylsuccinimide and maleic anhydride (VSI)m - (MAh)n (proreagent of the copolymer of maleic acid (VSI)m - (MA)n)

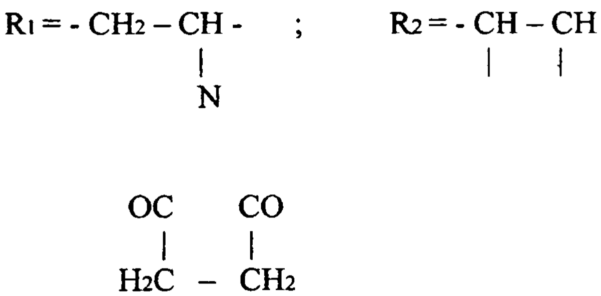

; n = 50

6) Copolymer of N-vinylpyrrolidone and maleic anhydride (VP)m - (MAh)n (proreagent of the copolymer of maleic acid (VP)m - (MA)n)

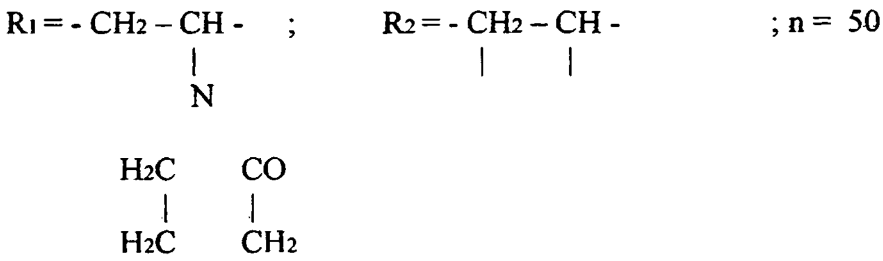

7) Polymer - polyacrylic acid (PAA)

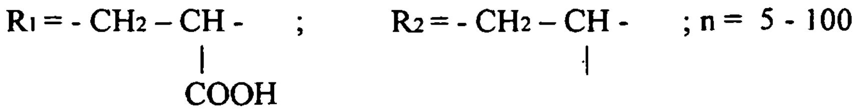

In copolymers of (VA)m - (VASA)n and (VP)m - (VASA)n the values m and n in accordance with the reaction capacity of monomers and used method of producing polymers can be in the range n = 1 - 99 and m = 100 - n . A noticeable antiviral activity of these copolymers starts with the values n ≥ 5, and an increase of n-values over 50 slightly influences the level of antiviral activity. That is why to ensure claimed properties of polymeric salts use of the value n = 5 - 50 is necessary and sufficient.

For the copolymers (VP)m - (MAh)n and (VSI)m - (MAh)n m = n = 50 mol%. These values are determined by the peculiarities of chemical structure and reaction capacity of monomers VP, VSI, MAh in reactions of their interpolymerization conditioning a strict sequence of copolymer -links and an equal number of units.

In copolymers (VP)m - (CA)n the range of values m and n is determined by the peculiarities of reaction capacity of monomers VP and CA during interpolymerization. In said range of formulations n = 5 - 15 (m = 85 - 95), a copolymer ensures production of salts with claimed biological properties.
The possibility of production of neutral and acid salts of PVASA and PAA with claimed properties is reflected by the values n = 5 - 100.

**Summary of the Invention**

The essence of the invention is explained by the information stated further, and namely:

- general method of synthesis of claimed salts;
- 7 examples of synthesis (for each of claimed salts);
- results of produced compounds identification;
- results of measurement of biological properties of synthesized salts (toxicity and antiviral activity).

**General method of synthesis of claimed salts**

The producing method for polymeric salts of 3.5-dimethyl-1-adamantylammonium is the interaction of a carboxylated (co)polymer in the aquatic medium with 3.5-dimethyl-1-adamantylamine at normal or reduced temperature (15 - 25 C°) with mixing of the components for 0.5 - 1 hour and admittance of the equimolecular ratios of reagents as calculated per actual or given carboxyl groups content in a (co)polymer and further recovery of salts from the solution by precipitation or using one of the spray or sublimation drying types.

A general initial reagent in the synthesis of polymeric salts of 3.5-dimethyl-1-adamantylamine can be produced and used as an intermediate product in the synthesis of its hydrochloride or can be recovered from commercial-grade hydrochloride (memantine) by its treatment with a 5% water solution of sodium hydrochloride and further extraction by diethyl ether or chloroform. Then ether (chloroform) is distilled and 3.5-dimethyl-1-adamantylamine is distilled under vacuum. Produced 3.5-dimethyl-l-adamantylamine does not change its biological activity and is a colorless oily liquid, insoluble in water but soluble in dimethyl sulfoxide, as well as in concentrated and diluted acetic and hydrochloric acid.

The pureness of produced 3.5-dimethyl-1-adamantylamine was assessed based on data of the element analysis and infrared spectroscopic analysis. The results of the element analysis of $C_{12}H_{21}N$:
Calculated, %: C=80.37; H=11.80; N=7.81

Found, %: C=80.41; H=11.95; N=7.70

As the second component - a polymeric base - compositionally homogeneous (by their chemical composition and molecular weight) carboxylated, water soluble, and non-toxic (co)polymers were selected:

1) polyvinylaminosuccinic acid (PVASA);
2) copolymer of vinyl alcohol and N-vinylaminosuccinic acid (VA)m - (VASA)n;
3) copolymer of N-vinylpyrrolidone and N-vinylaminosuccinic acid (VP)m - (VASA)n;
4) copolymer of N-vinylpyrrolidone and crotonic acid (VP)m - (CA)n;
5) copolymer of N-vinylsuccinimide and maleic anhydride (VSI)m - (MAh)n;
6) copolymer of N-vinylpyrrolidone and maleic anhydride (VP)m - (MAh)n;
7) polyacrylic acid (PAA).

The listed polymers can be produced according to the earlier method (A.F. Nikolaev et al. The Journal of Applied Chemistry, 1970, V.43, №6, pp. 1339 - 1343; A.F. Nikolaev et al. High-molecular Compounds, 1972, V. 14A, №11, pp. 2368 - 2370; 1974, B16, №1, pp. 14 - 16; S.N. Ushakov et al. High Molecular Compounds, 1967, A9, №8, pp. 1807 - 1810; T.P. Karpinskaya, E.M. Lukina, Plastic Masses, 1980, №6, pp. 14 - 15).

Molecular weight of used polymers is in the range of 20 - 80 thousand, the number of carboxylated links in co (polymers) is from 5 to 100 mol%.

Salt formation reaction of 3.5-dimethyl-l-adamantylamine with carboxylated polymers is implemented in the aquatic medium at room or reduced temperature (15 - 25 C°), with mixing of the components for 0.5 - 1 hour and admittance of the equimolar ratios of reagents as calculated per actual (or rated) carboxyl groups content in a (co) polymer with further recovery of salts from the solution by precipitation into acetone or methylethyl ketone, or the mixture of ethyl alcohol and diethyl ether, or the mixture of diethyl ether with acetic acid, or the mixture of diethyl ether with benzol, or using one of the spray or sublimation drying types.

Produced compounds are polymeric salts of polycarboxilic acids and 3.5-dimethyl-1-adamantylamine (3.5-dimethyl-1-adamantylammonium - (DMAAM). The composition and structure of produced salts were identified based on data of element analysis, infrared analysis, potentiometric titration in non-aquatic medium.

**Synthesis examples of claimed salts**

Example 1. Into a kettle (retort) equipped with a mixer 0.858 g of poly-N-vinylaminosuccinic acid (PVASA) and 30 $cm^3$ of distilled water are loaded. After polymeric solution 1.074 g of 3.5-dimethyl-1-adamantylamine (DMAAM) is admitted. The initial molar ratio of VASA:DMAAM is 1:1. The reaction takes place at the temperature of 15 C° during 1 hour, after that $30m^3$ of ethyl alcohol is poured into the reaction mixture and the product is produced by precipitation into the mixture of benzol and diethyl ether (ratio 1:3). Precipitation is filtrated, rinsed with diethyl ether and dried at 30 - 35 C°/6.65 Pa. Based on results of the elements analysis and potentiometric titration DMAAM content in a produced salt is 54.32 wt% which corresponds to the links content of the of 3.5-dimethyl-1-adamantylamine-ammonium salt of vinylaminosuccinic acid (VASA - DMAAM) in the amount of 95mol%. Product output (PVASA - DMAAM) constituted 89%. Characterizing viscosity of a polymeric salt solution in HCl concentration of c(HCl) 0.1 $mol/dm^3$ at 25 C° has the value:

$$[\eta]_{0.1MHCl} = 30\ cm^3/g.$$

Example 2. The process is similar to the one described in Example 1, but into a retort 1.0 g of vinyl alcohol copolymer with N-vinylaminosuccinic acid (VA - VASA) (VASA content is 25mol%, i.e. 52 wt%. 0.520 g) and $20cm^3$ of distilled water are loaded, and then a mixer is turned on. After complete polymer solution 0.650 g of 3.5-dimethyl-1-adamantylamine (DMAAM) is loaded. The initial equimolar ratio of VASA:DMAAM is 1:1. The process is implemented at room temperature (20 - 25 C°) during 0.5 hour until complete homogenization of aquatic reaction medium. The product is produced by precipitation into acetone; it is filtrated, rinsed with diethyl ether and dried at 30 - 35 C°/6.65 Pa. Based on the results of the elements analysis and potentiometric titration DMAAM content in a produced salt is 39.42 wt% which corresponds to 25 mol% of the VASA - DMAAM links (the links of 3.5-dimethyl-l-adamantylamine-ammonium salt of vinylaminosuccinic acid). Product output (VA - VASA - DMAAM) is 95%. Characterizing viscosity of a polymeric salt solution in HCl concentration c(HCl) 0.1$mol/dm^3$ at 25 C° constitutes:

$$[\eta]_{0.1MHCl} = 14\ cm^3/g.$$

Example 3. The process is similar to the one described in Example 1, but into a reaction retort 0.991 g of the copolymer of N-vinylpyrrolidone with N-vinylaminosuccinic acid (VP - VASA) with the VASA links content 27 mol% and 50 $cm^3$ of distilled water are loaded. After polymer solution 0.4 g of DMAAM is admitted. The reaction takes place at 20 C° during 40 minutes. The product is produced by lyophilization (sublimation drying) of the solution, is rinsed with diethyl ether and dried at 30 - 35 C°/6.65 Pa. Produced salt contains 28.75wt% of DMAAM corresponding to 27 mol% of the saline VASA - DMAAM links in the copolymer VP - VASA - DMAAM. Product output is 93%. The value of characterizing viscosity of a polymeric salt solution in HCl concentration c(HCl) 0.1 mol/$dm^3$ constitutes

$$[\eta]_{0.1MHCl} = 16\ cm^3/g.$$

Example 4. The process is similar to the one described in Example 1, but into the retort 1.212 g of N-vinylpyrrolidone with crotonic acid (VP-CA) copolymer and 40 $cm^3$ of distilled water are loaded. After polymer solution 0.2 g of DMAAM is admitted. The reaction takes place at the temperature of 20 C° during half-hour with mixing and further sedimentation of a resultant into ethyl alcohol. Recovered polymer is rinsed with diethyl ether and dried at 35 C°/6.65 Pa. Produced salt VP-CA-DMAAM contains (based on the analysis results) 10mol% of CA-DMAAM links which corresponds to complete interaction of DMAAM with carboxyl copolymer groups. Product output is 94%. The value of characterizing viscosity is

$$[\eta]_{0.1MHCl} = 12\ cm^3/g.$$

Example 5. The process is similar to Examples 1 - 4, but into the retort 0.5575 g of N-vinylsuccinimide copolymer with maleic anhydride of the content 50:50 mol% and 30 $cm^3$ of distilled water are loaded. The reaction mixture is mixed for 2 hours at 70 C° till complete hydrolysis of maleic anhydride links with formation of maleic acid. The temperature of the reacting mass is reduced down to 25 - 20 C° and 0.58 g of DMAAM is admitted. After full homogenization of water solution in 1 hour, the resultant is recovered by precipitation into the mixture of ethyl alcohol and acetone (1:3) and is filtered, rinsed with diethyl ether, and dried at 35 C°/6.65 Pa. Produced polymeric salt (based on the results of the elements analysis and potentiometric titration) contains 51 wt% of DMAAM. i.e. 70mol% of saline carboxylateammonium groups. Product (VSI-MA-DMAAM) output is 87%. The value of characterizing viscosity of the solution constitutes

$$[\eta]_{0.1MHCl} = 40\ cm^3/g.$$

Example 6. The process is similar to the one described in Example 5, but into the retort 0.5225 g of N-vinylpyrrolidone copolymer with maleic anhydride and 30 $cm^3$ of distilled water are loaded. After polymer solution (conditions are similar to Example 5) 0.4120 g of DMAAM is admitted. The resultant is recovered similar to Example 5. Produced polymeric salt contains 44.1 wt% of DMAAM which corresponds to cyclization of 3.5-dimethyl-1-adamantylammonium salt with 50 mol% of carboxyl copolymer groups. Product (VP-MA-DMAAM) output is 90%. The value of characterizing viscosity of the solution is:

$$[\eta]_{0.1MHCl} = 38\ cm^3/g..$$

Example 7. The process is similar to Examples 1 - 4, but into the retort 0.6 g of polyacrylic acid (PAA) and 30 $cm^3$ of distilled water are loaded. After polyacid solution, mixing, 0.4 g of DMAAM is admitted. The reaction takes place at 20 C° during 1 hour. The resultant is recovered by the sublimation drying of the solution. Dry product is rinsed with diethyl ether and dried at 30 C°/6.65 Pa. Polymeric salt PAA and DMAAM contains 40.1 wt% of DMAAM which corresponds to 27 mol% of the acrylate links of 3.5-dimethyl-1-adamantylammonium. i.e. production of acid salt PAA-DMAAM. Product output is 98%. The value of characterizing viscosity of the solution in 0.1 mol/$dm^3$ of HCl is

$$[\eta]_{0.1MHCl} = 17\ cm^3/g.$$

All produced polymeric salts of 3.5-dimethyl-1-adamantylammonium are white amorphous powders, soluble in water, in diluted acetic and hydrochloric acids, and in dimethyl sulfoxide. They are practically insoluble in 95% ethyl alcohol and diethyl ether. The values of characterizing viscosity of polymeric salt solutions in HCl water solution with the c(HCl) concentration 0.1 mol/$dm^3$ at 25 C° are in the range of 10 - 40 $cm^3$/g.

**Identification of produced compounds**

In the IR spectrum of dry polymeric compounds being the invention essence, absorption bands at 1560 - 1610 $cm^{-1}$ are observed as corresponding to valence vibrations of a C-O link of ionized group -COO; during formation of a salt link absorption bands at 800 $cm^{-1}$ are detected characterizing the presence of $N^+H_3$ groups of ammonium salt, and there is no absorption band at 1720 $cm^{-1}$ (or the intensity with regard to an initial polymer is reduced) corresponding to non-ionized -COOH groups in a polymer. In the IR spectrum of produced salts there are also absorption bands at 1350 $cm^{-1}$ and 1000 $cm^{-1}$ characteristic for an adamantan structure.

**Determination of biological properties of synthesized compounds**

**Determination of toxic properties of compounds**

The toxicity level of claimed compounds was assessed according to a general index of acute toxicity - average fatal dose of $LD_{50}$. The study was held with a single oral administration of the agent to white mice of both sexes, line "Balb", weight 18 - 20 g, and to white rats, line "Vistar", weight 130 - 250 g (female) and 225 - 370 g (male). The medications were administered to mice in the dose range from 250 to 1100 mg/kg, and to rats in the range of 480 - 2315 mg/kg.

It was determined that $LD_{50}$ values of claimed polymeric compounds DMAAM for mice are in the range from 445 to 806 mg/kg, and for rats - from 647 to 1180 mg/kg depending on the qualitative and quantitative composition of polymeric salts.

These results showed that claimed polymeric salts possess lower toxicity as compared to memantin ($LD_{50}$ for mice 363mg/kg) when the medication with an equimolecular dose of 3.5-dimethyl-1-adamantylamine) is administered to animals.

Synthesized salts are considered low-toxic compounds in accordance with the toxicity indices determined for mice and rats.

**Determination of antiviral properties of synthesized compounds**

The tests of antiviral activity of produced polymeric salts were conducted against the viruses of types A and B influenza using the tissue culture of chorionallantoic coat fragments from chick-embryos (model CAC) and infecting animals (mice). Test results are given in Tables 1 - 4.

*Table 1*

*Comparative characteristic of the antiviral activity of claimed polymeric salts and hydrochloride 3.5-dimethyl-1-adamantylammonium (HC-DMAAM) against the virus of the influenza of the type A/Victoria35/72 based on the CAC model*

| Example | Medication | Medication dose as calculated per equimolecular content of DMAAM, mcg/ml | Protection index of the medication, % |
|---|---|---|---|
| | HC - DMAAM (memantine) | 25.0 | 39 |
| 1 | PVASA - DMAAM | 25.0 | ≥90 |
| 2 | VA - VASA - DMAAM | 25.0 | ≥90 |
| 3 | VP - VASA - DMAAM | 25.0 | ≥90 |

(continued)

| *Comparative characteristic of the antiviral activity of claimed polymeric salts and hydrochloride 3.5-dimethyl-1-adamantylammonium (HC-DMAAM) against the virus of the influenza of the type A/Victoria35/72 based on the CAC model* | | | |
|---|---|---|---|
| Example | Medication | Medication dose as calculated per equimolecular content of DMAAM, mcg/ml | Protection index of the medication, % |
| 4 | VP-CA-DMAAM | 25.0 | 62 |
| 5 | VSI - MA - DMAAM | 25.0 | 72 |
| 6 | VP - MA - DMAAM | 25.0 | 75 |
| 7 | PAA - DMAAM | 25.0 | ≥90 |
| | Control (placebo) | 0 | 0 |

*Table 2*

| *Comparative characteristic of the antiviral activity of synthesized salts and DMAAM hydrochloride against the virus of the influenza A/Li/40 based on the CAC model* | | | |
|---|---|---|---|
| Example | Medication | Medication dose as calculated per equimolecular content of DMAAM, mcg/ml | Protection index of the medication, % |
| | HC - DMAAM (memantine) | 25.0 | 0 |
| 1 | PVASA - DMAAM | 25.0 | 35 |
| 2 | VA - VASA - DMAAM | 25.0 | 33 |
| 3 | VP - VASA - DMAAM | 25.0 | 32 |
| 4 | VP-CA-DMAAM | 25.0 | 20 |
| 5 | VSI - MA - DMAAM | 25.0 | 25 |
| 6 | VP - MA - DMAAM | 25.0 | 30 |
| 7 | PAA - DMAAM | 25.0 | 32 |
| | Control (placebo) | 0 | 0 |

*Table3*

| *Comparative characteristic of the antiviral activity of claimed polymeric compounds and hydrochloride DMAAM against the virus of the influenza B/Li/40 with a medical and preventive treatment scheme when administered to mice orally* | | | | | | | |
|---|---|---|---|---|---|---|---|
| Example | Medication | Single dose of the medication as calculated per equimolecular content of DMAAM, mg/ml | Administration frequency | The number of animals | | | Protection index of medication, % |
| | | | | Taken in the experiment | Died | | |
| | | | | | Animal units | % | |
| | HC - DMAAM | 50 | 5 | 20 | 13 | 65 | 7.1 |

(continued)

| Comparative characteristic of the antiviral activity of claimed polymeric compounds and hydrochloride DMAAM against the virus of the influenza B/Li/40 with a medical and preventive treatment scheme when administered to mice orally | | | | | | | |
|---|---|---|---|---|---|---|---|
| Example | Medication | Single dose of the medication as calculated per equimolecular content of DMAAM, mg/ml | Administration frequency | The number of animals | | | Protection index of medication, % |
| | | | | Taken in the experiment | Died | | |
| | | | | | Animal units | % | |
| 2 | VA - VASA - DMAAM | 50 | 5 | 20 | 7 | 35 | 50.0 |
| 3 | VP-VASA-DMAAM | 50 | 5 | 20 | 6 | 30 | 57.0 |
| 4 | VSI - MA - DMAAM | 50 | 5 | 21 | 11 | 52 | 25.7 |
| 5 | PAA - DMAAM | 50 | 5 | 20 | 10 | 50 | 28.6 |
| | Control (placebo) | 0 | - | 20 | 14 | 70 | - |

*Table 4*

| Comparative results of the duration of the antiviral activity of claimed compounds and hydrochloride DMAAM against the influenza A/Aichi/2/68 virus with a preventive application scheme when a single dose is administered to mice orally in the amount of 100mg/kg as calculated per equimolecular DMAAM content | | | | | | | |
|---|---|---|---|---|---|---|---|
| Example | Medication | Protection index of medication (%) when administered in specified periods (hours) before contamination | | | | | |
| | | 1 | 24 | 48 | 72 | 96 | 120 |
| | HC-DMAAM | 37 | 28 | 22 | 11 | 9 | 7 |
| 1 | PVASA - DMAAM | 63 | 55 | 50 | 50 | 35 | 35 |
| 2 | VA - VASA - DMAAM | 61 | 54 | 52 | 48 | 38 | 35 |
| 3 | VP - VASA - DMAAM | 60 | 55 | 53 | 45 | 35 | 30 |
| 6 | VP - MA - DMAAM | 60 | 52 | 40 | 28 | 22 | n/a |
| 7 | PAA - DMAAM | 53 | 40 | 33 | 20 | n/a | n/a |

In each experimental and control (placebo) group 20 mice of both sexes were infected.

The results of the experiments given in the Tables show that the known 3.5-dimethyl-l-adamantylamine hydrochloride (memantine) selected as an object for comparison of this invention demonstrates antiviral activity only against the influenza virus of A type. Against the influenza virus of B type memantine, as well as midantan and remantadin and their polymeric derivatives (USSR. Author's sertificates №№523618 and 640544), is not effective. In virology practice, an agent with the protection index less than 20 is considered inactive.

At the same time synthesized (claimed) polymeric compounds of 3.5-dimethyl-1-adamantylamine have an effective level of antiviral activity against the influenza virus of B type, both in the infection model of chick-embryo and mice.

In the experiments with use of the fatal infection of A influenza for mice, the synthesized polymeric salts when used according to the prevention scheme showed an effective, durable action with an effective protection up to 5 days after single contamination of animals, while with the use of memantine in the same dose as calculated per 3.5-dimethyl-1-adamantylamine (alkali) content, the medication was effective only when administered 24 hours before the animals were infected.

**Industrial use**

The information given in the sections "Summary of the invention" and "Determination of the biological properties of synthesized salts" proves that the synthesis of claimed salts is quite possible by the means described in the application. Produced compounds are expressly identified by IR analysis, elements analysis, and potentiometric titration methods; have high antiviral activity against viruses of influenza of A and B types; and at that differ by a durable (prolonged) action which allows using produced salts not only as agents with antiviral action for sparing treatment but for prevention of viral diseases.

Thus, claimed compounds comply with all the requirements to an invention - they are new, unobvious for a specialist (have an invention level) and are exploitable.

**Claims**

1. Polymeric salts of 3.5-dimethyl-1-adamantylammonium and water-soluble carboxylated (co)polymers of the general formula (1)

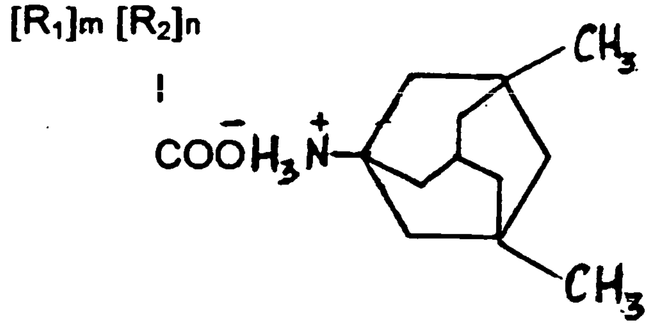

where **m** and **n** are mol%, **m** = **100 - n**,

and carboxylated (co)polymers are selected from the group:

polyvinylaminosuccinic acid,
copolymer of vinyl alcohol and N-vinylaminosuccinic acid,
copolymer of N-vinylpyrrolidone and N-vinylaminosuccinic acid,
copolymer of N-vinylpyrrolidone and crotonic acid;
copolymer of N-vinylsuccinimide and maleic anhydride;
copolymer of N-vinylpyrrolidone and maleic anhydride;
polyacrylic acid.

2. A substance as in claim 1, wherein polyvinylaminosuccinic acid (PVASA) is selected as a carboxylated polymer.

3. A substance as in claim 1, wherein a copolymer of vinyl alcohol and N-vinylaminosuccinic acid (VA)m - (VASA)n, where n = 1 - 99 mol%, and better n = 5 - 50 mol% is selected as a carboxylated polymer.

4. A substance as in claim 1, wherein a copolymer of N-vinylpyrrolidone and N-vinylaminosuccinic acid (VP)m - (VASA) n, where n = 1 - 99 mol%, and better n = 5 - 50 mol% is selected as a carboxylated polymer.

**5.** A substance as in claim 1, wherein as a carboxylated polymer, a copolymer of N-vinylpyrrolidone and crotonic acid (VP)m - (CA)n, where n = 5 -15 mol% is selected.

**6.** A substance as in claim 1, wherein a copolymer of N-vinylsuccinimide and maleic anhydride (VSI)m - (MAh)n, where n = 50 mol% is selected as a carboxylated polymer.

**7.** A substance as in claim 1, wherein a copolymer of N- vinylpyrrolidone and maleic anhydride (VP)m - (MAh)n, where n = 50 mol% is selected as a carboxylated polymer.

**8.** A substance as in claim 1, wherein polyacrilic acid (PAA) is selected as a carboxylated polymer.

**9.** Use of a substance as in claims 1 - 8 as an antiviral agent.

**10.** An antiviral agent wherein any of the substances from claims. 1 - 8 are selected as an active substance.

**Patentansprüche**

**1.** Polymere Salze von 3,5-Dimethyl-1-adamantylammonium und wasserlöslichen carboxylierten (Co)polymeren der allgemeinen Formel (I)

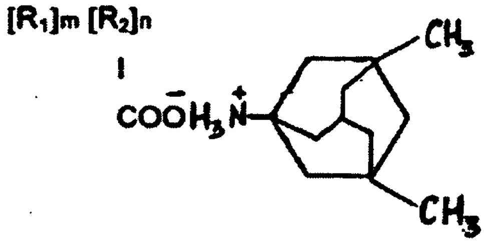

wobei m und n Mol-% sind, m = 100 - n ist,

und carboxylierte (Co)polymere aus der Gruppe ausgewählt sind bestehend aus:

Polyvinylaminobernsteinsäure, Copolymer von Vinylalkohol und N-Vinylaminobernsteinsäure, Copolymer von N-Vinylpyrrolidon und N-Vinylaminobernsteinsäure, Copolymer von N-Vinylpyrrolidon und Crotonsäure; Copolymer von N-Vinylsuccinimid und Maleinsäureanhydrid; Copolymer von N-Vinylpyrrolidon und Maleinsäureanhydrid; Polyacrylsäure.

**2.** Substanz nach Anspruch 1, wobei die Polyvinylaminobernsteinsäure (PVASA) als carboxyliertes Polymer ausgewählt ist.

**3.** Substanz nach Anspruch 1, wobei ein Copolymer von Vinylalkohol und N-Vinylaminobernsteinsäure (VA)m - (VASA) n, wobei n = 1 - 99 Mol-% und noch besser n = 5 - 50 Mol-% als carboxyliertes Polymer ausgewählt ist.

**4.** Substanz nach Anspruch 1, wobei ein Copolymer von N-Vinylpyrrolidon und N-Vinylaminobernsteinsäure (VP)m - (VASA)n, wobei n = 1 - 99 Mol-% und noch besser n = 5 - 50 Mol-% als carboxyliertes Polymer ausgewählt ist.

**5.** Substanz nach Anspruch 1, wobei als carboxyliertes Polymer ein Copolymer von N-Vinylpyrrolidon und Crotonsäure (VP)m - (CA)n, wobei n = 5 - 15 Mol-% ausgewählt ist.

**6.** Substanz nach Anspruch 1, wobei ein Copolymer von N-Vinylsuccinimid und Maleinsäureanhydrid (VSI)m - (MAh) n, wobei n = 50 Mol-% als carboxyliertes Polymer ausgewählt ist.

**7.** Substanz nach Anspruch 1, wobei ein Copolymer von Vinylpyrrolidon und Maleinsäureanhydrid (VP)m - (MAh)n, wobei n = 50 Mol-% als carboxyliertes Polymer ausgewählt ist.

**8.** Substanz nach Anspruch 1, wobei Polyacrylsäure (PAA) als carboxyliertes Polymer ausgewählt ist.

**9.** Verwendung einer Substanz nach den Ansprüchen 1 - 8 als antivirales Mittel.

**10.** Antivirales Mittel, wobei irgendeine der Substanzen aus den Ansprüchen 1 - 8 als aktive Substanz ausgewählt wird.

**Revendications**

**1.** Sels polymériques de 3,5-dimethyl-1-adamantyl-ammonium et (co)polymères carboxylés soluble dans l'eau selon la formule générale (I) :

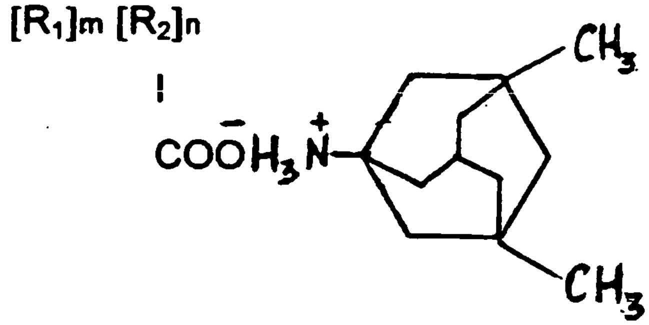

où m et n sont en % molaire, m=100-n,
et les (co)polymères carboxylés sont sélectionnés parmi le groupe :

- acide polyvinylaminosuccinique,
- copolymère d'alcool vinylique et d'acide N-vinylaminosuccinique,
- copolymère de N-vinylpyrrolidone et d'acide N-vinylaminosuccinique,
- copolymère de N-vinylpyrrolidone et d'acide crotonique,
- copolymère de N-vinylsuccinimide et d'anhydride maléique,
- copolymère de N-vinylpyrrolidone et d'anhydride maléique,
- acide polyacrylique.

**2.** Une substance selon la revendication 1, dans laquelle l'acide polyvinylaminosuccinique (APVAS) est sélectionné en tant que polymère carboxylé.

**3.** Une substance selon la revendication 1, dans laquelle un copolymère d'alcool vinylique et d'acide N-vinylaminosuccinique (VA)m-(AVAS), où n=1-99%mol, et mieux n=5-50%mol est sélectionné en tant que polymère carboxylé.

**4.** Une substance selon la revendication 1, dans laquelle un copolymère de N-vinylpyrrolidone et d'acide N-vinylaminosuccinique (VP)m-(AVAS), où n=1-99%mol, et mieux n=5-50mol% est sélectionné en tant que polymère carboxylé.

**5.** Une substance selon la revendication 1, dans laquelle en tant que polymère carboxylé, un copolymère de N-vinylpyrrolidone et d'acide crotonique (VP)m-(AC)n, où n=5-15%mol est sélectionné.

**6.** Une substance selon la revendication 1, dans laquelle un copolymère de N-vinylsuccinimide et d'anhydride maléique (VSI)m-(AhM)n, où n=50%mol est sélectionné en tant que polymère carboxylé.

**7.** Une substance selon la revendication 1, dans laquelle un copolymère de N-vinylpyrrolidone et d'anhydride maléique (VP)m-(AhM)n, où n=50%mol est sélectionné en tant que polymère carboxylé.

**8.** Une substance selon la revendication 1, dans laquelle l'acide polyacrylique (APA) est sélectionné en tant que polymère carboxylé.

**9.** Utilisation d'une substance selon l'une des revendications 1 à 8, en tant qu'agent antiviral.

**10.** Un agent antiviral dans lequel l'une quelconque des substances selon les revendications 1 à 8 est sélectionnée en tant que principe actif.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **M.D. MASHKOVSKI.** Medicinal Agents. *New Wave,* 2002, vol. 1, 143
- **V.A. SNEZHKO ; L.N. SAMOILOVA et al.** *Antibiotics Journal,* 1972, vol. 17 (1), 48-52
- **F.I. ERSHOV.** Antiviral Medications. *Medicine,* 1998, 141-142
- **A.F. NIKOLAEV et al.** *The Journal of Applied Chemistry,* 1970, vol. 43 (6), 1339-1343
- **A.F. NIKOLAEV et al.** *High-molecular Compounds,* 1972, vol. 14A (11), 2368-2370
- *HIGH-MOLECULAR COMPOUNDS,* 1974, vol. B16 (1), 14-16
- **S.N. USHAKOV et al.** *High Molecular Compounds,* 1967, vol. A9 (8), 1807-1810
- **T.P. KARPINSKAYA ; E.M. LUKINA.** *Plastic Masses,* 1980, (6), 14-15